# EUROPEAN PATENT APPLICATION

(11) **EP 4 799 613 A1**
(43) Date of publication of application: **02.09.2026**
(21) Application number: 24882440.1
(22) Date of filing: 24.10.2024
(51) Int. Cl.: A61K 9/22, A61K 31/155, A61K 47/04, A61K 47/10, A61K 47/32

(54) **PHARMACEUTICAL COMPOSITION AND TABLET**

(30) Priority: 27.10.2023 JP 2023184800
(71) Applicant: Mitsubishi Chemical Corporation, Tokyo 100-8251 (JP)
(72) Inventor: MORIOKA Toshifumi, Tokyo 100-8251 (JP); TANIGUCHI Yutaka, Tokyo 100-8251 (JP); YOSHIMURA Nobuyoshi, Tokyo 100-8251 (JP)
(74) Representative: Vossius & Partner Patentanwälte Rechtsanwälte mbB
(86) International application number: PCT/JP2024/037884
(87) International publication number: WO 2025/089329

(57) **Abstract**

Disclosed is a pharmaceutical composition providing a tablet capable of retaining a tablet shape over prolonged duration after being ingested, while the content of polyvinyl alcohol is suppressed.

The pharmaceutical composition comprises a polyvinyl alcohol-based resin (A), a polyhydric phenol compound (B), and amorphous silica (C). The content of the amorphous silica (C) ranges from 0.1 to 20 parts by mass based on 100 parts by mass of the polyvinyl alcohol-based resin. Also, disclosed is a tablet comprising the pharmaceutical composition as a matrix composition (I). and an active pharmaceutical ingredient (II) which is dispersed in the matrix composition (I), wherein the content of the active pharmaceutical ingredient (II) ranges from 30 to 90% by mass.

## Description

### TECHNICAL FIELD

The present invention relates to a pharmaceutical composition and a tablet using the same, and more specifically, relates to a pharmaceutical composition suitable for a matrix material used for sustained-release tablets and a tablet containing the same.

### BACKGROUND

A sustained-release tablet is controlled in release rate of an active pharmaceutical ingredient so as to extend the time of drug release over a long period. Such a sustained-release tablet may reduce frequency of administration and thereby improve medication compliance, moreover, may lessen fluctuations in blood concentration and thereby lower a side effect. Thus, the tablet has been actively studying and developing in recent years.

There are a various sustained-release tablets based on a variety of mechanism of controlling the drug release: a membrane permeation-controlled tablet including a sustained-release coating layer which can control permeation of the active pharmaceutical ingredient; a matrix diffusion-controlled tablet including insoluble substance as a main excipient; a matrix type tablet employing a hydrophilic polymer as a matrix material; an osmotic pressurecontrolled tablet; and so on.

Among these, the matrix type sustained-release tablet employing a hydrophilic polymer for the matrix material is advantageous in terms of manufacturing method as it can be produced by direct compression. Therefore, the formulation of the material has been actively studied in these days.

When the matrix type sustained-release tablet is exposed to a physiological medium, the hydrophilic polymer, which is a basic material of matrix, swells to form a mesh-like gel matrix, which prevents the rapid release of the drug. The physiological medium then gradually penetrates the interior of the tablet, leading to the gradual breakdown of the gel matrix, and eventually the tablet loses its shape. This process is referred to as tablet disintegration.

Polyvinyl alcohol (PVA) is a biodegradable hydrophilic polymer and is an effective candidate for the matrix material contained in matrix-type sustained-release tablets.

To extend the drug release time of matrix-type sustained-release tablets employing polyvinyl alcohol as the matrix material, it is necessary to prolong the disintegration time of the formed gel matrix.

However, when the amount of polyvinyl alcohol contained in the matrix material is low, the strength of the formed gel is insufficient, the sustained-release properties deteriorate, and the tablet becomes more susceptible to disintegration due to peristaltic movement in the digestive tract. These factors can lead to excessive drug release.

Conversely, increasing the amount of polyvinyl alcohol results in the formation of a gel having a dense network structure, which can delay the drug release time. Nevertheless, a higher content of polyvinyl alcohol also increases the tablet size. Since the tablet must meet a predetermined size requirement for proper dosing, the amount of polyvinyl alcohol that can be incorporated in a single tablet is therefore limited.

To achieve a desired sustained release by increasing the strength of the gel matrix, Patent Document 1 (WO2022/202138) proposes a polyhydric phenol compound (tannic acid) is incorporated into matrix material employing PVA.

Patent Document 1 further discloses that the combined use of tannic acid increases the tablet hardness and suppress the drug dissolution rate one hour and three hours after administration.

Furthermore, Patent Document 2 (WO2023/027056) discloses that desirable sustained-release properties were achieved by increasing tablet hardness through using a combination of polyvinyl alcohol and crystalline cellulose for a matrix material, and adjusting the average particle size and blend ratio of the combination to fall within a specific range.

### PRIOR ART

### PATENT DOCUMENT

[Patent Document 1] WO 2022/202138
[Patent Document 2] WO 2023/027056

### SUMMARY OF THE INVENTION

### TECHNICAL PROBLEM TO BE SOLVED BY THE INVENTION

Each of the sustained-release tablets disclosed in Example of the Patent Document 1 contain less than 50% by mass (approximately 47%) of metformin hydrochloride as an active pharmaceutical ingredient. Furthermore, the example discloses that 100% by mass of the active pharmaceutical ingredient was released within 10 hours in a dissolution test of metformin hydrochloride.

Nowadays, sustained-release tablets are required to retain their active pharmaceutical ingredients for at least 10 hours after administration so that they can be taken only once a day. To meet this demand, it is necessary to prolong the retention time of the matrix while adjusting the content of polyvinyl alcohol serving a basic material of the matrix, within a predetermined range.

Patent Document 2 discloses that the sustained-release tablets described in the examples contained less than 50% by mass (approximately 48%) of the active pharmaceutical ingredient (metformin hydrochloride), and that the dissolution rate was less than 100% even 10 hours after administration. However, nearly 50% of the drug was released in the early stage of administration (one hour after administration), indicating that there is still room for improvement in achieving a sustained-release tablet capable of maintaining its efficacy over a longer period.

The present invention has been made in consideration of the above circumstances, and its object is to provide a pharmaceutical composition that can provide sustained-release tablets containing 50% by mass or more of an active pharmaceutical ingredient per tablet using polyvinyl alcohol as a matrix material, or a sustained-release tablet that retains an amount of the active pharmaceutical ingredient after a dissolution test, and tablets comprising the pharmaceutical composition.

### MEANS FOR SOLVING THE PROBLEM

To provide a tablet containing 50% by mass or more of an active pharmaceutical ingredient with excellent sustained efficacy, releasing the active pharmaceutical ingredient even 10 hours after administration, the present inventors have conducted various studies on components that could increase gel strength while reducing the content of polyvinyl alcohol-based resin serving a matrix. They ultimately found that incorporating a combination of silica particles and a polyhydric phenol compound into the matrix allows the tablet to maintain its shape in a swollen state even 10 hours after administration, thereby completing the present invention.

According to one aspect of the invention, the pharmaceutical composition includes the following embodiments:
(1) A pharmaceutical composition comprising a polyvinyl alcohol-based resin (A), a polyhydric phenol compound (B), and amorphous silica (C), wherein the content of the amorphous silica (C) ranges from 0.1 to 20 parts by mass based on 100 parts by mass of the polyvinyl alcohol- based resin (A).
(2) The pharmaceutical composition of the embodiment (1), further comprising an active pharmaceutical ingredient (D), in a content of 30% by mass or more and 90% by mass or less.
(3) The pharmaceutical composition according to the embodiment (1) or (2), wherein the content of the polyvinyl alcohol-based resin (A) in the pharmaceutical composition ranges from 5 to 35% by mass.
(4) The pharmaceutical composition according to any one of the embodiments (1) to (3), wherein the polyhydric phenol compound (B) is contained in an amount of 0.05 to 32 parts by mass based on 100 parts by mass of the polyvinyl alcohol-based resin (A).
(5) The pharmaceutical composition according to any one of embodiments (1) to (4), wherein the specific surface area of the amorphous silica (C) measured by BET method ranges from 50 to 1000 m²/g.
(6) The pharmaceutical composition according to any one of embodiments (1) to (5), wherein the amorphous silica (C) is a chain-like aggregate of silica nanoparticles.
(7) The pharmaceutical composition according to any one of embodiments (1) to (6), wherein the polyvinyl alcohol-based resin (A) has an average degree of polymerization of 500 to 3,000 and a degree of saponification of 78 to 96 mol%.
(8) The pharmaceutical composition according to any one of embodiments (1) to (7), wherein the mass ratio (B/C) in contents of the polyhydric phenol compound (B) to the amorphous silica (C) ranges from 30/70 to 95/5.

According to another aspect of the invention, the present invention relates to a tablet.
(9) A tablet comprises the pharmaceutical composition of any one of the above embodiments (1) to (8). The tablet of the present invention involves the following embodiments:
(10) A tablet comprising a matrix composition (I) and an active pharmaceutical ingredient (II) dispersed in the matrix composition (I), wherein the matrix composition (I) comprises a polyvinyl alcohol-based resin (A), a polyhydric phenol compound (B), and amorphous silica (C), and wherein a remaining portion of the tablet after a dissolution test according to the Japanese Pharmacopoeia, has a loss tangent value of 10 or less. The loss tangent, tan δ, is defined as a ratio of the loss modulus (E") to the storage modulus (E'), determined from its dynamic viscoelasticity measurements.
(11) The tablet according to the embodiment (10), wherein the content of the active pharmaceutical ingredient (II) ranges from 30% by mass or more and 90% by mass or less.
(12) The tablet according to the embodiment (10) or (11), wherein the content of the polyvinyl alcohol-based resin (A) is from 5 to 35% by mass.
(13) The tablet according to any one of the embodiments (10) to (12), wherein the specific surface area of the amorphous silica (C) measured by the BET method is 50 to 1000 m²/g.
(14) The tablet according to any one of the embodiments (10) to (13), wherein the amorphous silica (C) is a chain-like aggregate of silica nanoparticles.
(15) The tablet according to any one of the embodiments (10) to (14), wherein the polyvinyl alcohol-based resin (A) has an average degree of polymerization of 500 to 3000 and a degree of saponification of 78 to 96 mol%.
(16) The tablet according to any one of the embodiments (10) to (15), wherein the mass ratio (B/C) of the content of the polyhydric phenol compound (B) to the content of the amorphous silica (C) ranges from 30/70 to 95/5.

### EFFECT OF THE INVENTION

Tablets comprising the pharmaceutical composition of the present invention exhibit high gel strength, such that the gel remaining as a tablet after dissolution test of the tablet has a loss tangent (tanδ) of 10 or less. The loss tangent is determined from viscoelasticity measured using dynamic viscoelasticity analyzer. Therefore, the pharmaceutical composition is useful as a matrix material for tablets, particularly sustained-release tablets. A tablet using the pharmaceutical composition as a matrix material can incorporate a high amount of active pharmaceutical ingredient within a limited size, ensuring ease of administration, while providing sustained release of the active pharmaceutical ingredient over an extended time.

### BRIEF DESCRIPTION OF THE DRAWINGS

FIG. 1 is a schematic diagram illustrating the dissolution behavior of a sustained-release tablet.
FIG. 2 is a schematic diagram illustrating the tablet shape used to determine the shape-retention percentage evaluated in the Example.

### EMBODIMENT FOR CARRYING OUT THE INVENTION

### [Composition for a pharmaceutical tablet]

A pharmaceutical composition of the present invention comprises a polyvinyl alcohol-based resin (A), a polyhydric phenol compound (B), and amorphous silica (C), and the content of amorphous silica (C) ranges from 0.1 to 20 parts by mass based on 100 parts by mass of the polyvinyl alcohol-based resin (A). Furthermore, the pharmaceutical composition may contain an active pharmaceutical ingredient (D) in an amount of 30% by mass or more and 90% by mass or less. Each component will be described below.

### (A) polyvinyl alcohol (PVA)-based resin

A polyvinyl alcohol (PVA)-based resin (A) is a resin obtained by saponification of a polyvinyl ester-based resin which is a polymer of vinyl ester-based monomers. The PVA-based resin is a resin mainly composed of a vinyl alcohol structural unit generated from saponification of the vinyl ester-based monomer. The PVA-based resin comprises the vinyl alcohol structural unit in an amount corresponding to its saponification degree, and a vinyl ester structural unit as an unsaponified portion.

The polyvinyl alcohol (PVA)-based resin (A) is not limited to an unmodified PVA-based resin consisting of a vinyl alcohol unit and a vinyl ester unit. A modified polyvinyl alcohol-based resin which is a saponified copolymer of vinyl ester-based monomer and a monomer copolymerizable with the vinyl ester-based monomer, may be used. Also, a post-modified PVA-based resin may be used. However, unmodified PVA-based resin is preferred. Regarding the modified PVA-based resin, its modification degree, which is correspondent to a content of structural units other than a vinyl alcohol unit and a vinyl ester structural unit, is less than 10 mol%, preferably 5mol% or less, more preferably 1 mol% or less.

Examples of the vinyl ester monomer include vinyl formate, vinyl acetate, vinyl propionate, vinyl valerate, vinyl butyrate, vinyl isobutyrate, vinyl pivalate, vinyl caprate, vinyl laurate, vinyl stearate, vinyl benzoate, vinyl versatate and the like. Of these vinyl esters, vinyl acetate is preferably used for a practical reason.

Examples of the copolymerizable monomer include olefins such as ethylene, propylene, isobutylene, α-octene, α-dodecene, and α-octadecene; hydroxy group-containing α-olefins such as 3-buten-1-ol, 4-penten-1-ol, 5-hexen-1-ol, 3,4-dihydroxy-1-butene and their derivatives such as acylated products; unsaturated acids such as acrylic acid, methacrylic acid, crotonic acid, maleic acid, maleic anhydride, itaconic acid, undecylenic acid, and salts, monoesters, or dialkyl esters thereof; nitriles such as acrylonitrile and metaacrylonitrile; amides such as diacetoneacrylamide, acrylamide, and methacrylamide; olefin sulfonic acids such as ethylene sulfonic acid, allyl sulfonic acid, methallyl sulfonic acid or salts thereof; alkyl vinyl ethers; vinyl compounds such as dimethylallyl vinyl ketone, N-vinylpyrrolidone, vinyl chloride, vinylethylene carbonate, 2,2-dialkyl-4-vinyl-1,3-dioxolane, and glycerin monoallyl ether; substituted vinyl acetates such as isopropenyl acetate and 1-methoxyvinyl acetate; vinylidene chloride, 1,4-diacetoxy-2-butene, 1,4-dihydroxy-2-butene, vinylene carbonate and the like. Such copolymerizable monomers may be used alone or in combination of two or more of them. The copolymerizable monomer is contained in an amount of less than 10 mol%, preferably 5 mol% or less, particularly preferably 1 mol% or less, based on the total amount of the polymer.

A preferable modified PVA-based resin is a copolymer in which an unsaturated acid, or its salt, monoester, or dialkyl ester is copolymerized as the copolymerizable monomer. A polyvinyl alcohol/acrylic acid/methyl methacrylate copolymer is particularly preferred as a modified PVA-based resin.

The vinyl ester-based monomer (optionally including a copolymerizable monomer) can be polymerized by a conventionally known method (bulk polymerization, solution polymerization, suspension polymerization, dispersion polymerization, emulsion polymerization, etc.). As a polymerization catalyst and a solvent used in the polymerization, a conventionally known one may also be used. In general, solution polymerization using a methanol solvent may be applied.

Saponification of the resulting vinyl ester-based polymer can also be carried out by a conventionally known method. Industrially, the vinyl ester polymer is dissolved in alcohol and saponified in the presence of an alkali catalyst. A common alkali catalyst includes hydroxides or alcoholates of alkali metals such as sodium hydroxide, potassium hydroxide, and sodium methylate.

The polyvinyl alcohol-based resin particles obtained by saponification are washed with a lower alcohol such as methanol, and then dried with hot air or the like in a continuous or batch manner. An appropriate drying temperature is commonly between 50°C and 150°C, preferably between 60°C and 130°C, particularly preferably between 70°C and 110°C. An unduly high drying temperature may cause a thermal deterioration of a PVA-based resin particle. An unduly low drying temperature extends a drying time. An appropriate drying time is generally from 1 to 48 hours, preferably from 2 to 36 hours. If PVA-based resin particles are subject to drying for an excessively long period, thermal degradation may occur. Conversely, if the drying time is insufficient, the PVA-based resin particles may remain inadequately dried, or a higher drying temperature may be required for the drying operation. The PVA-based resin is dried such that the solvent content remaining in the dried PVA-based resin is typically 10% by mass or less, preferably 5% by mass or less, and particularly preferably 1% by mass or less.

The PVA-based resin (A) has an average saponification degree of 78 to 96 mol%, which satisfies the requirement for partially saponified polyvinyl alcohol in the Japanese Standards for Food Additives (JPE). Preferably, the average saponification degree of 85 to 89 mol%, which complies with the requirements of both the European Pharmacopoeia (EP) and the United States Pharmacopoeia (USP).

PVA-based resin having an average saponification degree within the said range may exhibit high water solubility, so the PVA-based resin contained in the orally administrated tablet may form hydrogen bonds with hydroxyl groups in the polyhydric phenol compound and silica particles to create a hydrogel which contributes to sustained-release of an active pharmaceutical ingredient from the tablet. On the other hand, PVA-based resin having an unduly low degree of saponification exhibits reduced gel-forming capability due to the decreased number of hydroxyl groups, thereby resulting in unsatisfactory sustained-release properties. Herein the average degree of saponification refers to the value measured by a method conforming to JIS K 6726 (1994).

The average degree of polymerization of the PVA-based resin (A) is 500 or more, preferably 800 or more, 1000 or more, 1500 or more, or 2100 or more. The upper limit is preferably 3,000 or less, and more preferably 2,600 or less. When the average degree of polymerization is too low, the resin exhibits insufficient gel-forming capability, resulting in inadequate suppression of drug release. In particular, polyvinyl alcohol-based resins with an average degree of polymerization below 800, which are commonly used as a binder described later, tend to dissolve in water rather than form a gel after oral administration. Consequently, they cannot adequately inhibit release of the active pharmaceutical ingredient, making it difficult to achieve desired sustained release properties. On the other hand, if the average degree of polymerization is too high, the elasticity of the PVA-based resin particles increases, which may result in a decrease in compression moldability and tablet formability. The average degree of polymerization herein refers to number average degree of polymerization measured by a method in accordance with JIS K 6726 (1994).

The viscosity of aqueous solution having a concentration of 4% by mass of the PVA-based resin used as the matrix material is, preferably 6.0 mPa.s or more, more preferably in the range of 15.0 mPa.s to 70.0mPa.s, more preferably in the range of 20.0 mPa.s to 60.0mPa.s, at 20°C. The aqueous solution (4% by mass) having an unduly high viscosity is generally caused by a PVA-based resin having excessively high average polymerization degree. Therefore, a PVA-based resin exhibiting an excessively high measured viscosity of its aqueous solution tends to reduce moldability during compression or tablet pressing due to increased elasticity. Conversely, if the viscosity of the 4% by mass aqueous solution is too low, indicating that the average degree of polymerization of the PVA-based resin is insufficient, the strength and density of the hydrogel formed upon contact with a physiological medium tend to be reduced, resulting in tablets with inadequate sustained-release properties. Hereinafter, the viscosity of the 4% by mass aqueous solution at 20°C is the viscosity measured by a method in accordance with JIS K 6726 (1994).

In tablet manufacturing process, the polyvinyl alcohol-based resin (A) in the form of powder, which is composed of many fine particles, may be blended with other components. Alternatively, the polyvinyl alcohol-based resin (A) may be dissolved in water to be granulated, and the granulated product may be blended. From the viewpoint of a manufacturing method and productivity of the tablet, using it in the form of powder is preferable.

The PVA-based resin particles constituting powder have an average particle size of 40µm to 200µm, preferably 45 to 100µm, more preferably 45 to 80µm, and even more preferably 45 to 70µm. If the average particle size is excessively large, the powder in a packed state is likely to contain increased voids, which may result in tablets having reduced hardness. The average particle size refers to the median diameter determined from the particle size distribution (volume basis) measured using laser diffraction.

A preferable content of the polyvinyl alcohol-based resin (A) in the pharmaceutical composition ranges from 5 to 35% by mass. A tablet produced from the composition of the present invention contains active pharmaceutical ingredients in an amount of 30% by mass or more, preferably 40% by mass or more, and more preferably 50% by mass or more. This limits the content of the polyvinyl alcohol-based resin to the above-mentioned range.

### Polyhydric phenol compound (B)

Examples of the polyhydric phenol compound (B) include catechol, resorcinol, hydroquinone, pyrogallol, oxyhydroquinone, phloroglucin, tannic acid and the like. These may be used alone or in combination of two or more. Among these polyhydric phenols, tannic acid is preferred.

Tannic acid can be extracted from a variety of plant materials. For example, tannic acid can be extracted with water or ethanol from persimmon fruit, chestnut astringent skin, quincunx, gallnut, cod powder, leguminous tamarind seed coat, or mimosa bark. A preferable tannic acid is a tannic acid extracted from quincunx or gallnut which is listed in the 18th revision of the Japanese Pharmacopoeia. Both unrefined and refined tannic acid may be utilized, but refined tannic acid is preferably used.

Polyphenol compounds such as tannic acid have a high affinity for water, and can form a hydrogen bond with a hydroxyl group of PVA-based resin and with silanol groups (Si-OH) on the surface of silica particles described later when PVA-based resin is present. The hydrogen bond between them can promote the formation of a three-dimensional network structure in a tablet. When a tablet containing the composition is produced, a gel having a three-dimensional network structure can suppress the release of active pharmaceutical ingredients contained in the tablet, and as a result, a desirable sustained-release can be achieved.

The polyhydric phenol compound (tannic acid) having such a role is contained in an amount of preferably 0.05 to 32 parts by mass, more preferably 0.1 to 30 parts by mass, 1 to 25 parts by mass, or 5 to 15 parts by mass based on 100 parts by mass of the PVA-based resin (A). When the composition is used as a matrix material in a tablet, the polyhydric phenol compound can contribute to densifying the network structure of the gel matrix. On the other hand, it is effective to increase the content of the polyhydric phenol compound while reducing the content of the polyvinyl alcohol-based resin, but if the amount of tannic acid added is too high, the tablets tend to become colored.

Since polyhydric phenol (B) such as tannic acid is in powder form at room temperature, the polyhydric phenol may be used as it is. However, a granule prepared from a polyhydric phenol solution by spraying and being dried may also be used.

The particle size of tannic acid powder is generally in the range between 5 and 100µm, preferably between 10 and 50µm. The particle size of tannic acid is a particle size measured by a laser diffraction method.

### Amorphous Silica (C)

Amorphous silica (C) is an amorphous silica particle possessing silanol groups on its surface.

The type and method of production of the amorphous silica employed in the invention are not particularly limited. Both wet silica, such as precipitated silica or sol-gel derived silica, and dry silica, such as silica produced by combustion or arc methods, may be utilized.

Dry silica is amorphous silica (silicon dioxide) produced by reacting silicon tetrachloride with oxygen in a high-temperature flame and a typical example is fumed silica. Commercially available products, including the Aerosil (trademark) series, may be used as dry silica.

Wet silica is generally classified into precipitated silica and sol-gel derived silica. Precipitated silica can be obtained by neutralizing an alkaline aqueous solution of sodium silicate or a similar compound, followed by filtration to recover the amorphous silica, washing with water, and drying. Sol-gel derived silica is produced by neutralization reaction between sodium silicate and sulfuric acid in an acidic pH range, thereby causing aggregation while suppressing the growth of primary particles.

Commercially available precipitated silica products include, for example, Nipsil, Ultrasil, Carplex, Mizukasil, and Tokusil, and commercially available sol-gel derived silica (including silica gel) products include, for example, Silysia, Syloid, and Nipgel.

Both wet silica and dry silica are present in the form of aggregate (secondary particles) composed of primary particles (silica nanoparticles) having particle sizes ranging from several nanometers to several hundred nanometers, or in the form of aggregates formed by further aggregation of secondary particles.

The silica particle preferably has a specific surface area ranging from 50 to 1000 m²/g, more preferably 75 to 800 m²/g, even more preferably 100 to 500 m²/g, particularly preferably 120 to 450 m²/g, and even more preferably 150 to 400 m²/g. The specific surface area is determined by BET method.

The shape of amorphous silica (C) aggregates, the state of aggregation (i.e. pore volume, pore diameter, etc.), and the aggregation force of the primary particles vary depending on the type of silica, production method, production conditions, and the like. In general, dry silica aggregates are structure in which spherical primary silica particles are linked together in a chain-like, bead-like, or irregular shape. In dry silica aggregates, the primary silica particles are held together by cohesive forces that are weaker than those of wet silica. Wet silica aggregates tend to form spherical aggregates due to relatively stronger cohesive forces between primary silica particles. Accordingly, wet silica aggregates are often present as porous particles having pores that constitute an internal surface area, similar to silica gel. A preferred silica is chain-like aggregates consisting of silica nanoparticles, as such structures exhibit superior shape retention.

Generally, the specific surface area of chain-like aggregate tends to be smaller than that of spherical aggregates composed of porous particles such as silica gel. For this reason, compared to wet silica, dry silica tends to have larger pores and specific surface area when considered as an aggregate.

Silanol groups (Si-OH) are present on the surfaces of the silica particles described herein. These silanol groups contribute to increased density and mechanical strength of the resulting gel matrix by forming hydrogen bonds with the hydroxyl groups present in the PVA resin and in the polyhydric phenol compound (e.g. tannic acid) under physiological conditions. Accordingly, in the pharmaceutical composition of the invention, the release duration of the active pharmaceutical ingredient is prolonged as a result of the extended maintenance of the tablet's structural integrity. Thus, a pharmaceutical tablet produced from the pharmaceutical composition exhibits improved the sustained-release properties.

From the viewpoint of the formation of a hydrogel with a three-dimensional network structure through hydrogen bonds with the OH groups of the PVA molecules, the function and reaction between the polyhydric phenol compounds and silica appear similar. However, the gel obtained by combining PVA with tannic acid and silica could have a higher crosslink density than the gel obtained by combining PVA with a polyhydric phenol compound alone or the gel obtained by combining PVA with silica alone. This may be because the combination of the polyhydric phenol compounds and silica results in the formation of denser hydrogen bonds, thereby enhancing the durability of the network structure of the matrix formed from the PVA-based resin. On the other hand, if the pore size of the silica particles (aggregates) becomes too small, the formation of hydrogen bonds with the PVA-based resin may be hindered because the PVA-based resin is a relatively large molecule. In other words, silica may readily form hydrogen bonds with other components, such as polyhydric phenol compounds and PVA-based resins, and may contribute to increasing the degree of crosslinking and stabilizing the hydrogel due to the weaker cohesive force of the primary particles with its chain-like aggregate.

Silica particles can also act as a fluidizing agent. The presence of silica particles contributes to reduction of porosity and pore size of the tablet produced by dry tableting process. Silica particles may enhance compressibility of the composition, thereby achieving the production of tablets with high hardness.

The amorphous silica (C) is usually supplied in the form of a powder (silica primary particles or aggregates or agglomerates of primary particles) in the formulation process. Such powder has a particle size as a median diameter D₅₀ of 0.1µm or more, 0.5µm or more, 1µm or more, or 5µm or more, and 500µm or less, 200µm or less, 100µm or less, or 50µm or less. The median diameter refers to as an integrated value of 50% in the particle size distribution. Silica powder having such a particle size is blended with the other ingredients and compressed to obtain a tablet by a compression molding process. In the case of silica present as aggregates or agglomerates formed through weak cohesive forces, the aggregates or agglomerates may be broken down during the mixing operation and become dispersed within the mixture. Accordingly, a mixed powder as a pharmaceutical composition is obtained, in which the amorphous silica (C) is dispersed among the other components including PVA-based resin (A) and polyhydric phenol compound (B).

The median particle size D₅₀ is determined as the 50% integrated value in the particle size distribution (volume basis) acquired using a laser diffraction particle size distribution analyzer.

Besides, hydrophobic silica particles having hydrophobic surface can be obtained by chemically fixing compounds such as organosilicon compounds or silicone oils onto the surface of fumed silica particles through the reaction of silanol groups (Si-OH) present on the particle surfaces.

According to the invention, both hydrophobic silica and hydrophilic silica can be utilized as the amorphous silica (C), provided that the material satisfies the above-defined ranges of specific surface area and particle size. However, hydrophilic silica is preferred because it possesses more hydroxyl groups (OH) on the surface, which can form hydrogen bonds with the hydroxyl groups of polyvinyl alcohol molecules and polyhydric phenol compounds and contribute to the formation of a dense hydrogel.

The mass ratio (B)/(C) in contents of the polyhydric phenol compound (B) to the amorphous silica (C) ranges from 30/70 to 95/5, preferably 40/60 to 92/8, more preferably 50/50 to 90/10.

A matrix substrate comprising a PVA-based resin as a main component is required to swell readily and to undergo appropriate breakdown through dissolution. Therefore, an unduly high content of silica particles should be avoided. From this viewpoint, the content of silica (C) based on 100 parts by mass of the polyvinyl alcohol-based resin (A) is 0.1 parts by mass or more, preferably 1 part by mass or more, more preferably 1.5 parts by mass or more, and even more preferably 2 parts by mass or more, and 20 parts by mass or less, preferably 10 parts by mass or less, and more preferably 8 parts by mass or less.

### Active pharmaceutical ingredient (API) (D)

The pharmaceutical composition of the present invention may further contain an active pharmaceutical ingredient (D).

Examples of the active pharmaceutical ingredient (D) include antipyretic analgesic antiphlogistics, nutrient and tonic supplements, psychotropics, antidepressants, antianxiety drugs, hypnosedatives, anticonvulsants, CNS-acting drugs, brain metabolism improving agents, brain circulation improving agents, antiepileptic agents, sympathomimetic drugs, gastrointestinal drugs, acid suppressants, anti-ulcerogenic drugs, cough medicines, antiemetics, anapnoics, bronchodilators, allergic drugs, antihistamine agents, agents for dental administration or oral administration, cardiants, agents for cardiac arrhythmia, diuretics, hypertension drugs, vasoconstrictors, coronary vasodilators, peripheral vasodilators, blood coagulation inhibitors, hyperlipidemias agents, cholagogues, antibiotics, chemotherapeutic agents, diabetes drugs, osteoporosis drugs, antirheumatics, skeletal muscle relaxants, antispasmodics, hormonal agents, alkaloid drugs, sulfa drugs, arthrifuges, and antineoplastics.

The active pharmaceutical ingredients suitable for use in the invention include those requiring sustained release administration, particularly orally administered active pharmaceutical ingredients that are readily water-soluble. Active pharmaceutical ingredients known to exhibit poor compressibility are also suitably employed. A particularly suitable active pharmaceutical ingredient is metformin hydrochloride.

The content of the active pharmaceutical ingredient (D) in the pharmaceutical composition is preferably 30% by mass or more and 90% by mass or less, more preferably 80% by mass or less, and even more preferably 75% by mass or less.

The tablet containing the pharmaceutical composition of the present invention can retain a shape as a tablet for a long time, and therefore, can contain the active pharmaceutical ingredient at a concentration as high as the range mentioned above.

Furthermore, the content of the active pharmaceutical ingredient in a tablet made from the composition of the invention is preferably 30% by mass or more, more preferably 40% by mass or more, and even more preferably 50% by mass or more.

### Other Components (E)

The pharmaceutical composition of the present invention may contain various additives within a range (20% by mass or less, preferably 10% by mass or less of the composition), that does not impair the effects of the invention.

### Binder (E1)

A binder is incorporated to promote interparticle adhesion in granulation process, whether by dry or wet process, and to ensure adequate cohesion in tableting process by direct compression or wet tableting.

Examples of appropriate binders include dextrin, gum arabic, gelatin, hydroxypropyl starch, methyl cellulose, hydroxypropyl cellulose, hypromellose, pullulan, starch paste, and polyvinyl alcohol-based resins.

The amount of binder is selected according to the type of compound employed, and is typically in the range of 0.1 to 10 parts by mass, preferably 0.2 to 5 parts by mass, more preferably 0.5 to 3 parts by mass, based on 100 parts by mass of the composition used for granulation.

Polyvinyl alcohol-based resin to be used as a binder (referred to as "PVA-based resin for binder") is distinguished from polyvinyl alcohol-based resin (A) to be utilized for matrix at least in terms of average degree of polymerization. While the number average degree of polymerization of the polyvinyl alcohol resin for binder usually ranges from 100 to less than 800, and preferably from 200 to less than 500.

An average saponification degree of the PVA-based resin for binder may be in the range of 70 to 99 mol%, and usually 78 to 96 mol%, preferably 85 to 89 mol%, which is similar to the PVA-based resin (A) used for the matrix material.

A PVA-based resin for binder can employ a modified polyvinyl alcohol-based resin disclosed for the polyvinyl alcohol-based resin (A), which is a saponified copolymer including a copolymerizable monomer.

When PVA-based resin is used for the binder, an aqueous solution obtained by dissolving it in water is preferably used.

When a tablet contains PVA-based resin as a matrix material as well as binder, the tablet contains two or more types of polyvinyl alcohol-based resins differing in average polymerization degree. In this case, the overall average polymerization degree of the polyvinyl alcohol-based resins contained in the solid dosage form is calculated as the sum of the individual average polymerization degrees, each determined independently based on their contents.

In the case of containing two types of PVA-based resins, for instance, a matrix PVA-based resin (A) and a binder PVA-based resin, the average saponification degree and average polymerization degree may be calculated respectively by the following formulas, wherein SDm and Pm represent a saponification degree and polymerization degree of the matrix PVA-based resin respectively, and SDb and Pb represent a saponification degree and polymerization degree of the binder PVA-based resin respectively. Also, M and B represent the respective proportion of the matrix PVA-based resin and the binder PVA-based resin relative to the total amount of PVA-based resins, wherein M+B=1.$Average saponification degree = SDm \times M + SDb \times B$ $Average polymerization degree = Pm \times M + Pb \times B$

The content of the PVA-based resin for binder is typically at most about 5% by mass, usually 3% by mass or less, preferably 2.5% by mass or less, based on the total weight of PVA-based resins. Therefore, even if the binder PVA-based resin having an average polymerization degree less than 800 is contained, the measurement value of the average polymerization degree of the PVA-based resins contained in the composition for solid dosage form may be 500 or more.

### Lubricant (E2)

A lubricant may be added so as to improve fluidity, provide lubricity during compression in the die and during tablet ejection, and avoid sticking to the punch faces or die wall. In particular, when tableting by direct compression, this is useful for reducing friction between the powder and punches and dies during compression.

Examples of the lubricant include stearic acid, magnesium stearate, calcium stearate, talc, and the like.

### Others (E3)

In addition to the above additives, other excipients (crystalline cellulose, sugar alcohols (e.g., mannitol, erythritol, xylitol, sorbitol, maltitol), sugars (e.g., glucose, fructose, lactose, sucrose, trehalose, maltose, oligosaccharides), calcium phosphates, starches, sodium phosphates, and gelatin, etc.; disintegrants (carmellose calcium, carboxymethyl starch sodium, croscarmellose sodium, crospovidone, cellulose or its derivatives, and starch or its derivatives, etc.); pH adjusters (citric acid and its salts, phosphoric acid and its salts, carbonic acid and its salts, tartaric acid and its salts, fumaric acid and its salts, acetic acid and its salts, amino acids and their salts, succinic acid and its salts, lactic acid and its salts, etc.); flow agents (titanium oxide, stearic acid, corn gel, heavy anhydrous silicic acid, etc.); surfactants (phospholipids, glycerin fatty acid esters, polyoxyethylene fatty acid esters, sorbitan fatty acid esters, polyethylene glycol fatty acid esters, polyoxyethylene hydrogenated castor oil, polyoxyethylene alkyl ethers, sucrose fatty acid esters, sodium lauryl sulfate, polysorbates, sodium hydrogen phosphates, potassium hydrogen phosphates, etc.); coloring agents (iron sesquioxide, yellow ferric oxide, food yellow No.5, food yellow No.4, aluminum chelate, titanium oxide, talc, etc.); and sweeteners (saccharin, aspartame, acesulfame potassium, thaumatin, sucralose, etc.) may be used as needed.

### <Tablets and their manufacturing method>

The tablets of the present invention are tablets containing the pharmaceutical composition disclosed herein.

The tablet of the invention is a tablet in which an active pharmaceutical ingredient (II) is distributed in a matrix composition (I) containing a polyvinyl alcohol-based resin (A), a polyhydric phenol compound (B), and amorphous silica (C).

Tablets are generally produced by blending various ingredients and molding them either directly or after granulation. Molding may be carried out either by tablet compression or wet tableting. In tablet compression, the mixture is directly compressed into tablets. Wet tableting is performed by drying or cooling a wet mass. The tablet manufacturing process is not limited to a specific method, but compression molding is preferred because it allows a dry process. Moreover, hard tablets with fewer voids can be readily obtained through compression molding.

In the case of compression molding, the matrix composition (I) is a uniform powder mixture obtained by homogeneously mixing powders of a PVA-based resin (A), a polyhydric phenol compound (B), and amorphous silica (C). In this case, the amorphous silica (C) powder having an average particle size referred as median diameter D₅₀, 0.1µm or more, 0.5µm or more, 1µm or more, or 5µm or more, and 500µm or less, 200µm or less, 100µm or less, or 50µm or less, is used. The silica powder exhibits fluidizing effect to reduce the interparticle gaps in the matrix composition.

Examples of the compression process include direct powder compression (direct compression), semi-dry granule compression, granule compression, or an appropriate combination thereof, and any of these processes may be used.

Tableting is preferably carried out to ensure the active pharmaceutical ingredient (II) is uniformly distributed within the PVA-based resin (A) in the final tablet. A preferred manufacturing method involves first preparing granules containing the active pharmaceutical ingredient (II), followed by blending these granules with the powder of the matrix composition (I) and then compressing the mixture into tablets.

A mixture of the matrix composition (I) and the active pharmaceutical ingredient (II) may be used as a granulation composition and then granulated. If necessary, other additives may be added to the granulation composition and mixed, or the granulation composition may be blended with other additives prior to tableting.

Examples of the granulation process include a wet granulation, dry granulation, and spray-dry granulation. In wet granulation, a binder solution is added to the powder composition and then granulated. Another method may be kneading the mixed composition to form a wet mass before being granulated. In dry granulation, the powder composition is compressed in its dry state, and then a lump of the compressed composition is broken down, and then granulated. In spray-dry granulation, a slurry made from the composition and a large amount of water is sprayed and dried to form granules.

The solvent used for a binder is appropriately selected according to the type of compound of the binder. In the case of using a binder PVA-based resin, water can be used as a solvent.

As a granulator, a dry granulator, a basket-type extrusion granulator, a stirring granulator, a centrifugal tumbling granulator, a fluid bed granulator, spray drying granulator and the like may be used. The dry granulator is a machine for performing a dry granulation by applying a high pressure to a powder blend to create a lump, breaking down the lump to obtain powder having an appropriate particle size. The stirring granulator is a machine for granulation by mixing the powder ingredients with a stirring blade for several minutes, and adding a binder solution dropwise to perform granulation while stirring. The fluid bed granulator is a machine for granulation which comprises mixing powder ingredients fluidized with air to form a powder blend and spraying a binder solution with a nozzle in a convection or countercurrent flow to help the powder blend for agglomeration by spraying droplets, and then drying to enlarge the size of the agglomerate. The spray drying granulator is a machine for performing granulation by spray drying. Among these, a granulation process using a fluidized bed granulator is preferred from the viewpoint of easily obtaining granulated products with excellent compressibility and also from the viewpoint of productivity.

For tableting, a rotary tableting machine, a single punch tableting machine, or the like, which is commonly used in the pharmaceutical field, can be used.

The compression pressure (tableting pressure) in compression molding is preferably about 1kN or more, more preferably about 2kN or more, and tableting can be carried out at about 4kN or more, 10kN or more, or 20kN or more. The upper limit is preferably about 60kN or less, and more preferably about 50kN or less. The pharmaceutical composition of the invention may be powder form that exhibits excellent compressibility and can provide a raw material powder mixture with few voids. Therefore, even if the composition powder is tableted at the compression pressure as high as the pressure described above, tablets free from chips can be obtained.

The pharmaceutical tablet may be ellipsoidal, cylindrical, spherical, doughnut-shaped, or any other shaped. Moreover, the tablet may be coated with a film, according to necessity.

The tablet of the invention is a tablet comprising the matrix composition (I), the active pharmaceutical ingredient (II), and other ingredients (III) as described above, and is produced, for example, by the method described above, preferably by tablet pressing. Other components (E) listed in the pharmaceutical composition of the invention may be used as the other ingredient (III).

From the viewpoint of ease of oral administration, the volume of the tablet is preferably 1000 mm³ or less, more preferably 800 mm³ or less, and even more preferably 750 mm³ or less.

The tablets produced as described above generally have a hardness between 50 and 200N, depending on the shape and size of the tablet. Such high-hardness tablets are usually difficult to chew, and after ingestion, they swell when in contact with physiological media, forming a gel matrix with a dense cross-linked structure, making them suitable as sustained-release tablets.

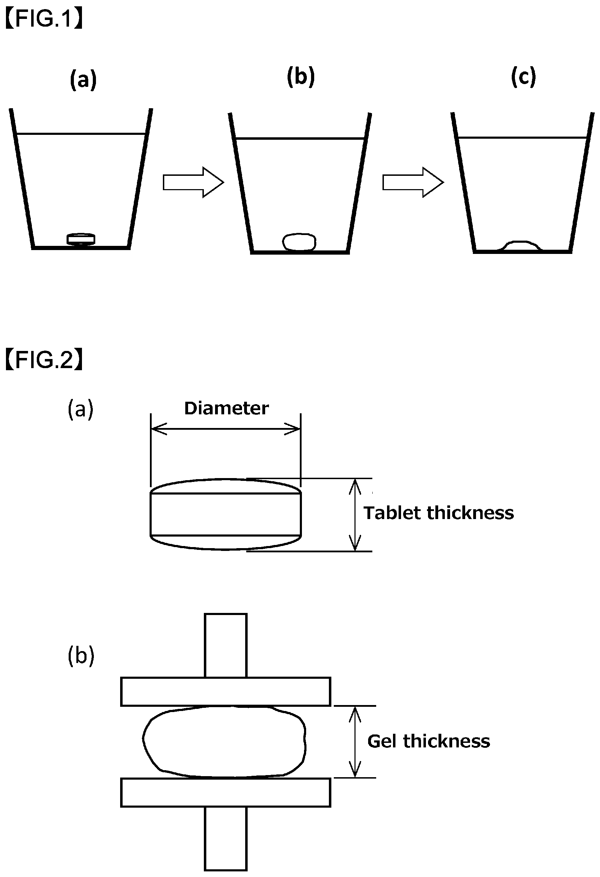

In a dissolution test on a tablet, the tablet is immersed in a solution corresponding to a physiological medium (Fig. 1(a)), the tablet absorbs the medium and swells to form a gel (see Fig. 1(b)), and the formed gel matrix dissolves and gradually erodes (Fig. 1(c)). In such way, the tablet is disintegrated while retaining part of its shape.

When the matrix composition (I) comes into contact with a physiological medium, such as intestinal fluid, the polyvinyl alcohol resin (A) forms a gel by establishing a three-dimensional network structure through hydrogen bonding with compounds possessing multiple hydroxyl groups, including polyhydric phenol compounds and silica particles. The hydrogel formed through hydrogen bonding between the polyhydric phenol compound and silica is stronger and more durable than the hydrogel formed from the PVA-based resin alone, although the reason is not clear. Accordingly, formed hydrogel can prolong the release time of the active pharmaceutical ingredient encapsulated within the matrix, resulting in excellent sustained release properties. The higher the degree of polymerization of the PVA-based resin, the more complex the entanglement structure with the tannic acid and silica particles may be formed, which results in improved sustained release properties. According to the invention, the tablet containing the disclosed pharmaceutical composition can form a gel with a long-lasting effect after administration and can release the active pharmaceutical ingredient over a long period of time. This means the tablet exhibits excellent sustained-release properties.

Specifically, the tablet has a loss tangent (tanδ) of 10 or less, preferably 8 or less, more preferably 5 or less, even more preferably 2 or less, and particularly preferably 1 or less, with respect to the remaining portion of the tablet after a dissolution test in accordance with the 18th Edition of the Japanese Pharmacopoeia, 6.10, The tanδ is defined as the ratio of the loss modulus (E") to the storage modulus (E'), as determined from measurements using dynamic viscoelasticity analyzer.

The dissolution medium and dissolution time to be used in the dissolution test are prescribed by the Pharmacopoeia in accordance with the type of drug to be evaluated. The tablet disclosed herein is distinguished in that its matrix composition is capable of imparting to the tablet a loss tangent, tanδ, falling within the above-mentioned range, irrespective of the dissolution medium and dissolution time specified for the particular drug contained therein. The loss tangent (tanδ) refers to as the ratio of loss modulus E" to storage modulus E', calculated from dynamic viscoelasticity measurement result for the remaining portion of the tablet after the above-mentioned dissolution test.

The tablet of the invention makes it possible to adjust the release rate, release time and release site of the active pharmaceutical ingredient from the tablet in accordance with the 18th revised Japanese Pharmacopoeia, for the purpose of reducing frequency of administrations or lowering side effects.

The gastric residence time of a tablet is typically approximately 2 to 3 hours. Accordingly, in order to maintain the drug concentration in the blood within a desirable range, it is preferable that the drug exhibits a low level of dissolution within one hour after administration and undergo controlled release over a period of 3 to 10 hours.

In the case of the tablets of the invention, even when the active pharmaceutical ingredient is contained at a high level of 50% by mass or more, the dissolution rate is suppressed to approximately 20 to 40% in the early stage of administration (i.e. one hour after initiation of the dissolution test). Thereafter, 85% or more of the active pharmaceutical ingredient is dissolved approximately 10 hours after administration (i.e. ten hours after initiation of the dissolution test).

The required release characteristics may vary depending on the type of drug. When the drug is metformin hydrochloride, the tablets of the invention satisfy either Rank A or Rank B, as specified in USP41 "Metformin Hydrochloride Extended-Release Tablets", indicating that the tablets exhibit the desired sustained release properties. Ranks specified in USP41 are shown below.

### Rank A:

Dissolution rate of active pharmaceutical ingredients 1 hour after initiation of the test: 20% to 40%.
Dissolution rate of active pharmaceutical ingredients 3 hours after initiation of the test: 45% to 65%.
Dissolution rate of active pharmaceutical ingredients 10 hours after initiation of the test: 85% or more.

### Rank B:

Dissolution rate of active pharmaceutical ingredients 1 hour after initiation of the test: 20% to 42%.
Dissolution rate of active pharmaceutical ingredients 3 hours after initiation of the test: 45% to 69%.
Dissolution rate of active pharmaceutical ingredients 10 hours after initiation of the test: 85% or more.

### Rank C:

Dissolution rate of active pharmaceutical ingredients 1 hour after initiation of the test being more than 42%, or 3 hours after initiation test being more than 69%.

### EXAMPLES

The present invention will be further explained below with reference to examples and comparative examples, but the present invention is not limited to the following examples.

### [Measurement and evaluation method]

### (1) Tablet hardness (N)

A hardness of a tablet manufactured by compression was measured using a PC-30 hardness tester (manufactured by OKADA SEIKO.CO., LTD.).

### (2) Dissolution test

Dissolution test was performed in accordance with the 18th revised Japanese Pharmacopoeia, 6.10.

Dissolution Test Fluid 2 (Fujifilm Wako Pure Chemical Industries, Ltd.), which complies with the Japanese Pharmacopoeia, was degassed at 45°C for 2 hours. One litter of the degassed medium, measured using a graduated cylinder, was transferred to a thermostatic, water-bath type dissolution tester (Model NTR-6600, Toyama Sangyo Co., Ltd.). After confirming that the test medium had reached 37°C, a single tablet was used to initiate the dissolution test. The dissolution rate was measured at 1 hour, 3 hours, and 10 hours after the dissolution test began.

The dissolution rate was determined in accordance with the procedure described in "Metformin Hydrochloride Extended-Release Tablets" of the USP (The United States Pharmacopeia). In this method, the amount of metformin hydrochloride present in the eluate obtained from the dissolution test was quantified by UV detection. Specifically, the content of metformin hydrochloride was calculated based on the ratio of the peak area attributable to metformin hydrochloride in a standard solution to that in the dissolution test solution. Assuming that a single tablet contains 500mg of metformin hydrochloride, the dissolution rate (%) is obtained by calculating the proportion of metformin hydrochloride detected in the dissolution test solution relative to this nominal content. When the calculated amount of metformin hydrochloride in the dissolution test solution is 500mg, the dissolution rate defined as 100%.

### (3) Dynamic viscoelasticity measurement

The remaining tablet (i.e. the remaining portion of the tablet) was picked up ten hours after the initiation of the dissolution test (2), and the loss tangent tanδ (loss modulus E"/storage modulus E') was determined from the measurement using a dynamic viscoelasticity analyzer (Type: Rheogel-E4000, UBM Co., Ltd.) according to the test conditions below.

### (Test conditions)

Measurement frequency: 0.05 Hz
Measurement strain: 5%
Chuck distance: Adjusted according to sample size
Deformation measurement mode: Compression
Measurement temperature: 22°C

### (4) Shape retention as a tablet structure

The shape retention percentage was calculated as the ratio of the thickness of the gel remaining as a tablet (i.e. the portion of the tablet remaining ten hours after initiation of the dissolution test (2)) to the thickness of the molded tablet depicted in Figure 2 (a). Shape retention was assessed as the percentage of the tablet structure retained after a dissolution test. The thickness of the gel was measured as the distance between the chucks holding the gel as depicted in FIG. 2(b), the chucks being a part of a dynamic viscoelasticity analyzer (Rheogel-E4000, UBM Corporation) employed for the measurement. $Shape retention \left(%\right) = gel thickness / tablet thickness \times 100$
○: Shape retention percentage of 60% or more
△: Shape retention percentage of 40% or more but less than 60%
X: Shape retention percentage of less than 40%

### (5) Particle size of raw material powder

The median diameter (D₅₀) of the powder was determined from the particle size distribution (volume basis) measured using a laser diffraction particle size analyzer (Model Mastersizer 3000, Malvern Panalytical Ltd).

### <Compounds used in tablet production>

### Matrix composition (I)

### PVA for matrix material (A)

Unmodified polyvinyl alcohol, with average saponification degree of 88 mol% and average polymerization degree of 2500, manufactured by Mitsubishi Chemical Corporation, was employed for PVA as matrix material (A).

### Polyphenol compound (B)

Tannic acid (refined product with alcohol) with median diameter of 33.8 µm, and manufactured by Kishida Chemical Co., Ltd., was employed for polyphenol compound (B).

### Amorphous silica (C)

- Silica 1: "AEROSIL 200" manufactured by Nippon Aerosil Co., Ltd., which is silica powder with a median diameter of 14.2µm, and individual particle of the powder is chain-like aggregate of spherical silica particles (hydrophilic), with specific surface area of 200m²/g.
- Silica 2: "AEROSIL 300" manufactured by Nippon Aerosil Co., Ltd., which is silica powder with a median diameter of 12.8µm, and individual particle of the powder is chain-like aggregate of spherical silica particles, with specific surface area of 300m²/g.
- Silica 3: "AEROSIL 50" manufactured by Nippon Aerosil Co.,Ltd., which is silica powder with a median diameter of 11.4µm, and individual particle of the powder is a chain-like aggregate of spherical silica particles, with specific surface area of 50m²/g.
- Silica 4: "SYLOSPHOBIC 200" manufactured by Fuji Silysia Chemical Industry Ltd., which is silica powder with a median diameter of 2.9µm, and individual particle of the powder is sol-gel derived silica particle with hydrophobic-treated surface, and with specific surface area of 220m²/g.

### Active pharmaceutical ingredient (II)

Metformin hydrochloride was used as the active pharmaceutical ingredient (II).

### Others (III)

- PVA for binder: Polyvinyl alcohol, with average saponification degree of 88 mol% and average polymerization degree of 300, manufactured by Mitsubishi Chemical Corporation
- Crystalline cellulose: "PH102" manufactured by Asahi Kasei Chemicals Corporation
- Magnesium stearate: Japanese Pharmacopoeia magnesium stearate manufactured by NOF Corporation

### [Tablet production]

### Tablets No. 1 to 7:

3.8g of PVA for binder was dissolved in 15.1g of purified water to prepare an aqueous solution of PVA-based resin. This PVA-based solution and 296 g of metformin hydrochloride were fed into a vertical mixing type granulator (FM-VG-01, Powrex Corporation), and wet granulation was carried out for 1 minute. The obtained granules were dried using a tumbling fluidized bed granulator (FD-MP-01, Powrex Corporation) to prepare metformin hydrochloride-containing granules with median diameter 170µm and moisture content 0.4%.

63.3g of the metformin hydrochloride-containing granules, 34.1g of PVA for matrix material, 3.4g of tannic acid, and silica powder indicated in Table 1 were fed in a plastic bag and mixed by vigorously shaking for 5 minutes. Furthermore, 1.0g of magnesium stearate was added and gently mixed. The resulting mixture was tableted at 35kN using a rotary tableting machine (HT-EX12SS-U manufactured by Hata Iron Works Co., Ltd.) to produce round tablets as depicted in FIG. 2(a). The tablet had a diameter of 14.2 mm and thickness of 5.9 mm, and a content of metformin hydrochloride of 500 mg.

### Tablet No. 8:

A tablet was produced in the same manner as No. 1, except that it did not contain silica powder.

The results of the above-mentioned measurement and evaluation methods for the sustained-release tablets are shown in Table 1.

### Tablet No. 9:

Tablets were produced in the same manner as No. 1, employing crystalline cellulose in place of silica particles. The result of the above-mentioned measurement and evaluation methods for the obtained sustained-release tablets are shown in Table 1.

**[Table 1]**

| No | | | 1 | 2 | 3 | 4 | 5 | 6 | 7 | 8 | 9 |
|---|---|---|---|---|---|---|---|---|---|---|---|
| Formulation (parts) | API-containing granule | | 63.3 | 63.3 | 63.3 | 63.3 | 63.3 | 63.3 | 63.3 | 63.3 | 63.3 |
| | PVA for matrix material | | 34.1 | 34.1 | 34.1 | 34.1 | 34.1 | 34.1 | 34.1 | 34.1 | 27.6 |
| | Tannic acid | | 3.4 | 3.4 | 3.4 | 3.4 | 3.4 | 3.4 | 3.4 | 3.4 | 2. 8 |
| | Silica 1(hydrophilic, 200m²/g) | | 0.5 | 1 | 2 | - | - | - | - | - | - |
| | Silica 2 (hydrophilic, 300m²/g) | | - | - | - | 0.5 | 1 | - | - | - | - |
| | Silica 3 (hydrophilic, 50m²/g) | | - | - | - | - | - | 0. 5 | - | - | - |
| | Silica4(phydrophobic, 220m²/g) | | - | - | - | - | - | - | 1 | - | - |
| | Crystalline cellulose | | - | - | - | - | - | - | - | - | 7. 1 |
| | Magnesium stearate | | 1.0 | 1.0 | 1.0 | 1.0 | 1.0 | 1.0 | 1.0 | 1.0 | 1.0 |
| Silica amount (parts) /100parts of PVA | | | 1.4 | 2.9 | 5.7 | 1.4 | 2.9 | 1.4 | 2. 9 | - | - |
| Tablet | Content of API (%) | | 61.1 | 60.8 | 60.2 | 61.1 | 60.8 | 61.1 | 60.8 | 61.4 | 61.4 |
| | Hardness (N) | | 117 | 137 | 168 | 138 | 167 | 98 | 73 | 46 | 47 |
| | Volume (mm³) | | 707.5 | 707.4 | 717.9 | 714.1 | 714.2 | 720.1 | 760.3 | 715.7 | 709.2 |
| | Density (mg/mm³) | | 1.15 | 1.16 | 1.16 | 1.15 | 1.16 | 1.15 | 1.09 | 1.14 | 1.15 |
| | Tan δ after completion of dissolution test | | 0.6 | 0.3 | 0.2 | 0.4 | 0.2 | 1.8 | 1.7 | 2.3 | 3.2 |
| Evaluation | Dissolution rate (%) | 1 hr | 39 | 37 | 37 | 36 | 38 | 35 | 34 | 40 | 43 |
| | | 3 hr | 67 | 62 | 61 | 59 | 61 | 61 | 58 | 72 | 68 |
| | | 10hr | 100 | 96 | 90 | 92 | 97 | 94 | 88 | 94 | 93 |
| | Shape retention (%) 10hr after initiation of dissolution test | | 66 | 100 | 113 | 71 | 88 | 41 | 44 | 35 | 16 |
| | Shape retention | | ○ | ○ | ○ | ○ | ○ | Δ | Δ | × | × |

Nos. 1 to 7, which were tablets containing silica, exhibited high tablet hardness and shape retention, as compared with Tablet Nos. 8.and 9, each of which did not contain silica. Also, Nos. 1 to 7 had a tendency that drug release was suppressed in the early stages of administration (one hour and three hours after administration), and satisfied the sustained release property evaluated as ranks A and B, while Nos. 8 and 9 exhibited the sustained release property evaluated as rank C.

When the same type of silica was used, shape retention improved as the silica content increased. For instance, in the case of containing Silica 1, shape retention increased in the order of No.1, No.2, and No.3, and in the case of containing Silica 2, shape retention increased in the order of No.4 and No.5.

Increased silica content reduces tan δ, which may indicate that silica enhances the crosslink density of the hydrogel and contributes to maintaining the solid state of the tablet.

All of Nos. 1, 4, and 6 had the same silica content and the same mixing ratio with tannic acid. Comparison between them, indicated that the larger the specific surface area, the smaller the tan δ tended to be. The results can be seen that the larger the specific surface area of chain-like silica, the more hydrogen bonds can be formed with other components including matrix PVA and tannic acid, to enhance the crosslink density of the formed gel.

From the comparison between Nos. 2, 5, and 7, No.7 had lower hardness, a larger volume and a lower shape retention. Furthermore, although No.2 and No.7 had similar silica contents and specific surface areas, No.7 exhibited significantly lower shape retention. No.7 employed silica particles whose surfaces were hydrophobized. The silica particle with hydrophobized surface may interfere the formation of hydrogen bonds with other components, as a result, the crosslink density of the formed gel may decrease.

### INDUSTRIAL APPLICABILITY

Tablets containing the pharmaceutical composition of the present invention exhibit sufficient hardness to resist chewing and maintain their tablet shape over an extended period after ingestion, thereby enabling sustained release of the active pharmaceutical ingredient over an extended duration. Accordingly, the pharmaceutical composition of the present invention can be used as a raw material to produce tablets that offer high patient compliance and are suitable for use as sustained-release dosage forms.

## Claims

1. A pharmaceutical composition comprising a polyvinyl alcohol-based resin (A), a polyhydric phenol compound (B), and amorphous silica (C),
wherein the content of the amorphous silica (C) ranges from 0.1 to 20 parts by mass based on 100 parts by mass of the polyvinyl alcohol-based resin (A).

2. The pharmaceutical composition according to claim 1, further comprising an active pharmaceutical ingredient (D) in an amount of 30% by mass or more and 90% by mass or less in the pharmaceutical composition.

3. The pharmaceutical composition according to claim 1 or 2, wherein the content of the polyvinyl alcohol resin (A) in the pharmaceutical composition ranges from 5 to 35% by mass.

4. The pharmaceutical composition according to any one of claims 1 to 3, wherein the polyhydric phenol compound (B) is contained in an amount of 0.05 to 32 parts by mass based on 100 parts by mass of the polyvinyl alcohol resin (A).

5. The pharmaceutical composition according to any one of claims 1 to 4, wherein the amorphous silica (C) has a specific surface area of 50 to 1000 m²/g as measured by BET method.

6. The pharmaceutical composition according to any one of claims 1 to 5, wherein the amorphous silica (C) is a chain-like aggregate of silica nanoparticles.

7. The pharmaceutical composition according to any one of claims 1 to 6, wherein the polyvinyl alcohol resin (A) has an average degree of polymerization of 500 to 3,000 and a degree of saponification of 78 to 96 mol %.

8. The pharmaceutical composition according to any one of claims 1 to 7, wherein the mass ratio (B/C) of the content of the polyhydric phenol compound (B) to the content of the amorphous silica (C) ranges from 30/70 to 95/5.

9. A tablet comprising the pharmaceutical composition according to any one of claims 1 to 8.

10. A tablet comprising a matrix composition (I) and an active pharmaceutical ingredient (II) dispersed in the matrix composition (I),
wherein the matrix composition (I) comprises a polyvinyl alcohol resin (A), a polyhydric phenol compound (B), and amorphous silica (C),
and wherein a remaining portion of the tablet after a dissolution test according to Japanese Pharmacopoeia 6.10. exhibits loss tangent (tanδ) of 10 or less wherein the loss tangent is a ratio of loss modulus E" to storage modulus E', as measured with dynamic viscoelasticity.

11. The tablet according to claim 10, wherein the content of the active pharmaceutical ingredient (II) is 30% by mass or more and 90% by mass or less.

12. The tablet according to claim 10 or 11, wherein the content of the polyvinyl alcohol resin (A) ranges from 5 to 35% by mass.

13. The tablet according to any one of claims 10 to 12, wherein the amorphous silica (C) has a specific surface area ranging from 50 to 1000 m²/g, as measured by BET method.

14. The tablet according to any one of claims 10 to 13, wherein the amorphous silica (C) is a chain-like aggregate of silica nanoparticles.

15. The tablet according to any one of claims 10 to 14, wherein the polyvinyl alcohol resin (A) has an average degree of polymerization of 500 to 3000 and a degree of saponification of 78 to 96 mol%.

16. The tablet according to any one of claims 10 to 15, wherein the mass ratio (B/C) of the content of the polyhydric phenol compound (B) to the content of the amorphous silica (C) ranges from 30/70 to 95/5.
